# FASCICULE DE BREVET EUROPEEN

(11) **EP 0 837 140 B1**
(45) Date de publication et mention de la délivrance du brevet: **14.08.2002**
(21) Numéro de dépôt: 97402419.2
(22) Date de dépôt: 14.10.1997
(51) Int. Cl.: C12P 19/44

(54) **Procédé de production de sophorolipides par fermentation cyclique avec alimentation en esters d'acides gras ou en huiles**
Verfahren zur Herstellung von Sophorolipiden durch zyklische Fermentation mit Zuführung von Fettsäureestern oder Ölen
Process for the production of sophorolipids by cyclic fermentation with fatty acid esters or oil supply

(30) Priorité: 18.10.1996 FR 9612797
(43) Date de publication de la demande: 22.04.1998
(73) Titulaire: INSTITUT FRANCAIS DU PETROLE, 92852 Rueil-Malmaison Cedex (FR)
(72) Inventeur: Marchal, Rémy, 78400 Chatou (FR); Warzywoda, Michel, 92500 Rueil Malmaison (FR); Chaussepied, Bernard, 78170 la Celle Saint Cloud (FR)

(56) Documents cités:
- DD-A- 252 002
- DD-A- 254 959
- FR-A- 2 670 798
- FR-A- 2 692 593
- WILLIAM C. MCCAFFREY ET AL.: "Sophorolipids production by Candida bombicola using self-cycling fermentation." JOURNAL OF FERMENTATION AND BIOENGINEERING, vol. 79, no. 2, 1995, pages 146-151, XP002054166
- ANNE-MARIE DAVILA ET AL.: "Kinetics and balance of a fermentation free from product inhibition:sophorose lipid production by Candida bombicola" APPL MICROBIOL BIOTECHNOL, vol. 38, no. 1, 1992, pages 6-11, XP002054167

## Description

L'invention concerne un procédé de fermentation cyclique avec alimentation du substrat (cyclic fed batch culture) pour la production d'une composition de sophorolipides.
Les sophorolipides sont par exemple utilisés en cosmétologie, comme agent antiradicalaire et agent antiélastasique (WO 95/34282). Ils peuvent être aussi utilisés comme co-tensioactif dans un procédé de dépollution de sols (EP-A 605 308).
Il a été mentionné dans les brevets US 3205150, US 3312684 et EP-B 516803 qu'une quantité de sophorolipides était produite par un procédé de fermentation mettant en jeu une culture de *Candida bombicola*.
Les sophorolipides sont considérés comme étant un mélange de composés représentés par les formules (1) et (2), dans laquelle R¹ représente l'hydrogène ou le groupe acétyle et R² l'hydrogène ou un groupement alkyle comportant 1 à 9 atomes de carbone, ou bien R² représente l'hydrogène ou un groupement méthyle, lorsque R³ est un chaîne hydrocarbonée insaturée à 13 ou 17 atomes de carbone.

Ces composés sont utilisables comme agents de nettoyage et comme émulsifiants et ils présentent d'excellentes propriétés hygroscopiques et hydrophiles dues à la partie sophorose de chacune de ces molécules et des propriétés hydrophobes provenant des parties acides gras.

La préparation de sophorolipides est généralement effectuée en présence d'un substrat tel que décrit dans le brevet US 3205150. Par exemple, ce substrat peut être des hydrocarbures, des acides gras saturés ou insaturés, des esters d'acides incluant des glycérides, des huiles végétales telles que l'huile de soja.

L'alimentation en substrat consiste à réaliser en discontinu (batch) à des intervalles de temps d'environ 12 à 24 heures, l'injection d'une quantité d'environ 2 % en poids par rapport au volume réactionnel initial, pour chaque addition. L'alimentation en substrat est plus avantageusement réalisée en continu (fed batch) comme dans le cas du brevet européen de la Demanderesse EP-B 516803.

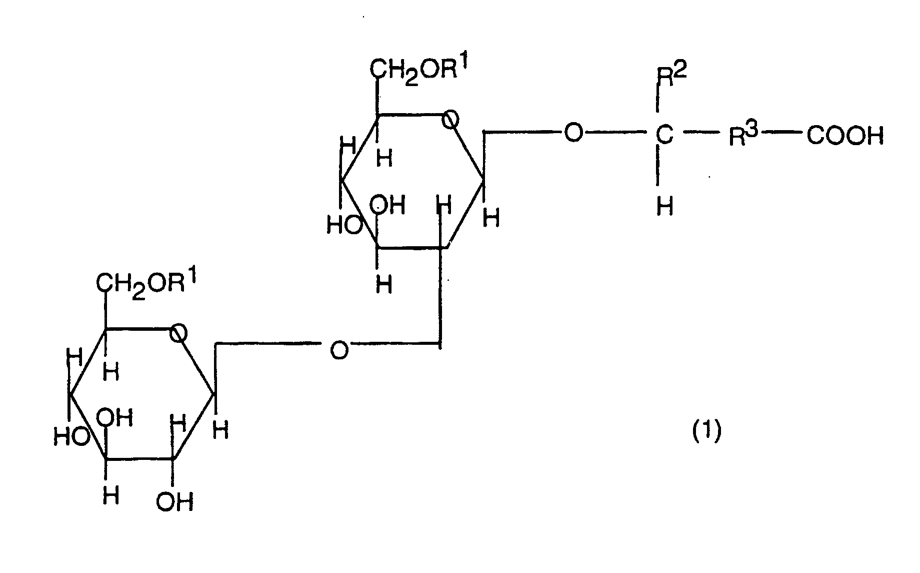

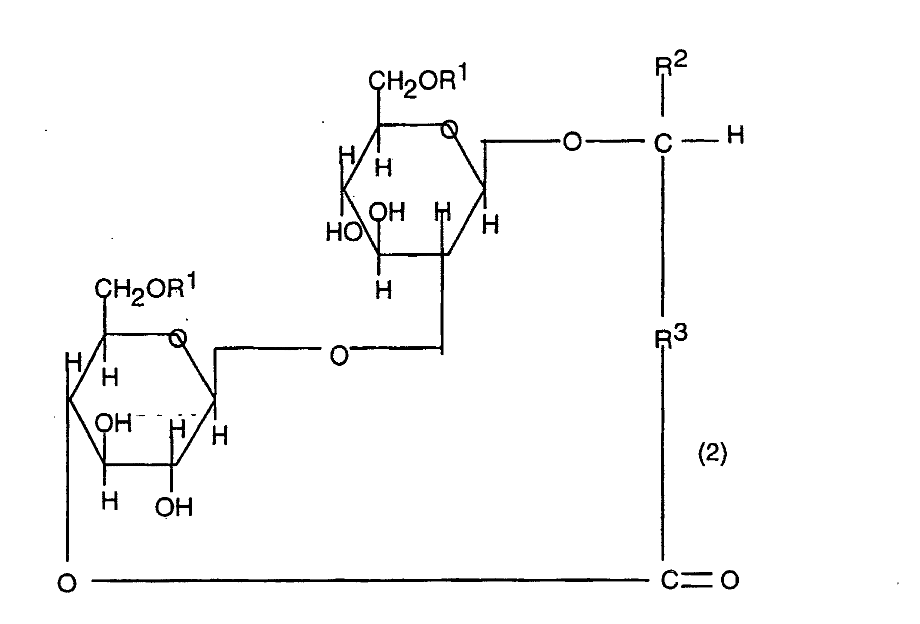

Structure des sophorolipides
(1) : formes acides
(2) : formes lactones

Les procédés de fermentation discontinus avec l'alimentation de substrat discontinue (batch) ou continue (fed batch) présentent les inconvénients classiques de la fermentation discontinue. Ainsi, la mise en oeuvre répétée à l'échelle industrielle d'une sérié d'opérations discontinues (en batch ou en fed batch) impose de nombreux temps morts dans le fonctionnement des installations en raison du nettoyage et de la stérilisation du fermenteur qui doivent être répétés entre chaque culture. De même, doit-on effectuer à chaque fois, la propagation de la souche productrice depuis son état de conservation en tube gélosé par exemple, jusqu'au fermenteur de production, par une chaîne d'ensemensement qui implique la culture successive du microorganisme dans des fermenteurs de tailles croissantes. Cette opération de préculture peut prendre plusieurs jours.

Pour éviter ces inconvénients et ces contraintes, on a recours habituellement à la culture continue dans laquelle on alimente le réacteur en milieu de culture et en substrat à débit équivalent et on soutire le moût fermenté à un débit équivalent. Le volume réactionnel est alors constant. Classiquement, la culture continue est réalisée selon le mode du chémostat (marque déposée) ( Wang et al. 1979. Continuous culture. In Fermentation and enzyme technology, John Wiley & Sons (eds), pp 98-137). Un nutriment du milieu de culture est présent en quantité limitante de façon à contrôler la concentration stationnaire du microorganisme dans le fermenteur et obtenir un fonctionnement stable du système. Lorsque l'on veut produire des métabolites (tels que l'éthanol par exemple), on utilise généralement deux ou plusieurs fermenteurs placés en série (système bi ou multi étages). Dans le premier est réalisée la croissance du microorganisme (premier étage de fermentation) avec un milieu comportant un élément nutritif limitant (azote, phosphore) tandis que l'excrétion de métabolites est réalisée dans le second réacteur dans lequel est admis le moût de fermentation du premier étage. On a observé de manière tout à fait inattendue que la souche placée dans ces conditions de production, perdait progressivement et rapidement ses propriétés d'excrétion et que la productivité était très inférieure à celle observée en fed batch.

Il a été également étudié, dans Chem. Abstr, vol. 122, n°15, 10 avril 1995, n° 185450, un procédé de fermentation relatif à la production de sophorolipides, dit "self cycling fermentation (SCF)" au cours duquel, au terme d'une période de temps correspondant au temps de croissance de la souche, on soutire la moitié du moût de fermentation obtenu que l'on remplace par du milieu frais (voir J. Ferment. Bio Eng. 1995, 79(2), p. 146, col. 1, 1. 11 avant la fin, associé au document Chem. Abstr.).

Il est préconisé d'allonger la durée de cycle après la période de croissance de la souche (doubling time) de 2 heures environ, de sorte que chaque nouveau cycle ne dépasse guère 6 heures. En opérant ainsi, on maintient certes la capacité de production de la souche mais la production de sophorolipides est très faible et leur récupération est alors très délicate à moins de les extraire par un solvant, ce qui complique le procédé et le rend encore plus coûteux.

D'autres documents tels que Chem. Abstr, vol. 119, n° 3, 19 juillet 1993, n° 26744, les demandes de brevet FR-A.2 670 798 et FR-A. 2 692 593 décrivent un procédé de production en fedbatch de sophorolipides comportant un cycle de fermentation.

Enfin, les demandes de brevet DD-A-252 002 et DD-A-254 959 concernent des procédés de fermentation cycliques pour la production de protéines, de stéroïdes et d'antibiotiques par des champignons et des bactéries, ces procédés ne décrivant ni ne suggérant de production de glycolipides, produits très différents de ceux mentionnés ci-haut, à partir de levures.

Un objet de l'invention est de remédier aux inconvénients mentionnés ci-avant. Un autre objet de l'invention est d'améliorer la productivité du système tout en obtenant des sophorolipides les plus purs possible de façon à éviter une étape d'extraction par solvant des produits obtenus. Un autre objet est d'accroitre la durée de production de sophorolipides dans les essais de fermentation qui dépassent la durée des essais discontinus (en batch ou fed batch), c'est à dire plus de 180 heures, tout en assurant des productivités élevées.

On a découvert un procédé de préparation de sophorolipides permettant d'allonger la durée de production et la productivité volumique de sophorolipides. Ce procédé met en oeuvre une technique particulière de culture d'une levure, la fermentation cyclique (Moser A 1985. Special cultivation techniques. In Biotechnology vol 2, Rehm HJ and Reed G (eds), pp 311-347).

Plus précisément, l'invention concerne un procédé de production d'une composition de sophorolipides comprenant la mise en culture dans une zone de réaction dans des conditions de culture aérée d'une souche *Candida bombicola* ou *Candida apicola* dans un milieu contenant une source d'azote et un substrat. Le procédé est caractérisé en ce qu'il comprend un premier cycle de fermentation comportant une étape de croissance de la souche et une étape de production d'un moût de fermentation contenant des sophorolipides au moyen d'une alimentation de la souche en substrat dans la zone de réaction. Le procédé est en outre caractérisé en ce qu'on effectue au moins une fois les étapes suivantes :
a) on soutire une partie du moût de fermentation;
b) on ajoute à la partie restante du moût un milieu minéral contenant de l'azote de façon à constituer un second milieu;
c) on effectue un nouveau cycle de fermentation à partir de ce second milieu et on produit un autre moût de fermentation.
La durée desdits premier et nouveau cycles de fermentation est d'au moins 30 heures. On récupère ensuite les sophorolipides du moût des étapes (a) et (c).

On a remarqué que dans ces conditions, on améliorait la productivité de la fermentation tout en obtenant une pureté de produits remarquable, c'est à dire sans présence d'une quantité substantielle d'acides gras ne nécessitant donc pas de fractionnement ultérieur pour les éliminer.
Cet avantage est d'autant plus marqué que l'alimentation des nouveaux cycles de fermentation est réalisée en continu.

On a par ailleurs constaté un gain considérable sur la chaîne d'ensemencement puisqu'on peut répéter la séquence de fermentation (étapes (a) à (c)) une pluralité de fois, par exemple 7 fois sans que la productivité en sophorolipides ne soit substantiellement affectée.

La durée de chaque cycle de fermentation sera en général d'au moins 30 heures et de préférence d'au moins 70 heures, par exemple 80 à 130 heures pour permettre d'une part à la souche de se multiplier suffisamment et d'autre part une accumulation de sophorolipides suffisante pour une bonne décantation.

Selon une caractéristique avantageuse du procédé, on peut séparer, par exemple par décantation, le moût de fermentation produit et récupérer un moût de fermentation débarrassé au moins en partie des sophorolipides sur lequel on effectue les étapes (a) à (c).

On améliore alors la pureté des produits c'est à dire que la quantité d'acides gras résultant de l'hydrolyse du substrat est telle qu'elle ne nécessite pas de fractionnement ultérieur pour les éliminer.

Selon une autre caractéristique, après avoir soutiré une partie du moût, on peut remplacer cette partie soutirée du moût par une quantité sensiblement égale de milieu minéral.

Avantageusement, on peut soutirer une quantité de moût telle qu'il reste au moins 5 % en poids, avantageusement 15 à 45 % et de préférence 20 à 40 % du moût dans la zone de réaction.

Selon une autre caractéristique du procédé, on peut interrompre chaque cycle de fermentation lorsque la productivité en sophorolipides durant le dit cycle diminue. De préférence on l'interrompt avant 140 heures de fermentation et plus particulièrement après une période de temps comprise entre 70 et 120 heures.

On stoppe généralement le dernier cycle de fermentation, c'est à dire la fermentation, lorsque la productivité en sophorolipides calculée sur les 24 premières heures de production de ce dernier cycle atteint 70 % de la productivité du premier cycle calculé sur la même période de production.

Selon une caractéristique particulièrement avantageuse permettant de minimiser la présence d'acides gras dans le moût obtenu, on peut maintenir la concentration résiduelle en substrat dans la zone de réaction durant chaque cycle de fermentation à une valeur qui n'excède pas 18 grammes par litre de volume réactionnel initial pendant qu'on alimente la zone de réaction en substrat.

C'est en alimentant la souche de préférence sensiblement en continu à un débit d'alimentation compris entre 0,01 et 4 grammes par heure et par litre de volume réactionnel initial que l'on peut contrôler au mieux cette concentration résiduelle en substrat et obtenir les meilleurs résultats en termes de pureté.

Une alimentation discontinue par petits ajouts en général inférieurs à 10g/l n'est toutefois pas à exclure.

Le substrat généralement utilisé est au moins une huile animale, au moins une huile végétale et/ou au moins un ester de ladite huile, ladite huile ou ledit ester incorporant une chaîne linéaire aliphatique de 10 à 24 atomes de carbone.

Avec la souche *Candida bombicola* CBS 6009, on a obtenu d'excellents résultats en termes de croissance et de production de sophorolipides. Le milieu de culture généralement choisi ainsi que le milieu minéral apporté lors de l'étape (b) peuvent contenir un sucre, par exemple du glucose.

L'invention sera mieux comprise au vu des exemples suivants :

### EXEMPLE 1 (selon l'invention) Culture cyclique avec alimentation continue et sans décantation du moût

La souche *C. bombicola* CBS 6009 est utilisée pour ensemencer un milieu de culture qui, indépendamment du glucose, est dépourvu de substrat. Le substrat est ajouté en continu dès le début de la culture. Il s'agit de l'ester éthylique de colza.

La composition du milieu de culture utilisé est la suivante :

| | |
|---|---|
| Glucose | 100 g.l⁻¹ |
| (NH₄)₂SO₄ | 4 g.l⁻¹ |
| KH₂PO₄ | 1 g.l⁻¹ |
| MgSO₄, 7H₂O | 0,5 g.l⁻¹ |
| Liqueur séchée de macération de maïs | 5 g.l⁻¹ |

Glucose et MgSO4, 7H2O sont stérilisés dans un fermenteur de laboratoire de 4 litres de capacité en solution dans 1620 ml d'eau tandis que KH₂PO_{4,} (NH₄)₂SO₄ et liqueur séchée de macération de maïs sont stérilisés séparément dans une fiole d'Erlenmeyer en solution dans 180 ml d'eau. La stérilisation des deux solutions est réalisée à l'autoclave à la température de 120°C pendant 30 minutes et le milieu de culture est reconstitué après refroidissement des deux solutions. Il est ensemencé par 200 ml d'une préculture préparée dans une fiole de Fernbach avec le même milieu que le milieu de fermentation mais additionné de 10 ml d'ester éthylique de colza. Cette fiole est ensemencée par 1 g environ d'un congelat de la souche de *C. bombicola*. Elle est incubée sous agitation à la température de 25°C. Elle fournit après 24 heures la préculture de fermenteur.
La fermentation de production est conduite dans un fermenteur de quatre litres de capacité sur un volume réactionnel initial de deux litres. L'agitation du milieu est obtenue avec une turbine RAYNERI (marque déposée) dont la vitesse de rotation est de 1000 rpm. L'aération est fixée à 0,5 vol/vol/minute d'air sous pression atmosphérique. Après auto-acidification du milieu de la culture, le pH est maintenu à la valeur constante de 3,5 grâce à une solution de soude 4N.
En cours de fermentation, on procède à des additions journalières de glucose sous forme solide à raison de 100 g aux temps 24, 48 et 72 heures. L'alimentation en ester éthylique de colza est réalisée dès le début de la culture avec une pompe péristaltique dont la vitesse d'alimentation est fixée à 2,1 g. par heure. On suit par des prélèvements d'échantillons la production de sophorolipides que l'on dose par la méthode de Göbbert et al. (Biotechnol. Lett. 1984, 6, 225-230). La production qui commence après la phase de croissance augmente avec l'addition de l'ester éthylique de colza. Après 96 heures de culture, on soutire une partie du moût fermenté de façon à ne laisser qu'un litre de moût dans le fermenteur. On complète celui-ci à deux litres avec un litre de milieu frais contenant du glucose à la concentration de 100 g.l⁻¹. On réalise un deuxième cycle de fermentation d'une durée de 96 heures avec le même débit d'ester de colza. On effectue alors la même opération de soutirage et de remplissage qu'à la fin du cycle précédent. On effectue ainsi au total 7 cycles de fermentation de 96 heures. On a donc fourni à la culture 500 g de glucose au premier cycle et 400 g à chacun des cycles suivants. La fourniture d'ester éthylique de colza est de 201,6 g à chaque cycle.

Les sophorolipides sont récupérés à partir des différents prélèvements effectués à chaque fin de cycle ainsi que sur le prélèvement final constitué par la totalité du moût de fin de fermentation. Sur chaque prélèvement, on laisse décanter les sophorolipides pendant une heure et on les récupère. On les remet en suspension sous agitation avec 1,5 litre d'eau distillée à la température de 45°C et on les laisse décanter à nouveau pendant quatre heures. On effectue un second lavage à 45°C dans des conditions identiques à celles du premier. On récupère ainsi à l'issue du second lavage un échantillon de sophorolipides hydratés sur lequel on mesure la teneur en eau par la méthode de Karl-Fisher, la teneur en sophorolipides anhydres par la méthode de Göbbert et al. (Biotechnol. Lett.1984, 6, 225-230) et la teneur en acides gras par la méthode de Davila et al. (Appl. Microbiol. Biotechnol. 1992, 38, 6-11). Les acides gras constituent, en effet, la principale impureté du produit. On déduit de ces mesures les productivités volumiques par rapport au volume initial (2 1) et les rendements massiques par rapport aux deux sources de carbone ajoutées, le glucose et l'ester éthylique de colza (Tableau 1)

**Tableau 1 :**

| Performances de la fermentation cyclique avec alimentation continue d'ester et sans décantation du produit | | | | | |
|---|---|---|---|---|---|
| Cycles | Heures des prélèvements | sophorolipides anhydres (g) | Acides gras dans sophorolipides (%) | Productivités (g.l⁻¹.h⁻¹) | Rendements |
| 1 | 96 | 155 | 0,5 | 0,8 | 0,22 |
| 2 | 192 | 255 | 0,5 | 1,33 | 0,42 |
| 3 | 288 | 240 | <0,5 | 1,25 | 0,4 |
| 4 | 384 | 265 | 0,8 | 1,38 | 0,44 |
| 5 | 480 | 270 | 0,5 | 1,4 | 0,45 |
| 6 | 576 | 230 | <0,5 | 1,2 | 0,38 |
| 7 | 672 | 450 | 0,7 | 2,34 | 0,74 |
| Moyennes | | 266 | 0,5 | 1,38 | 0,43 |

Cet exemple montre que la mise en oeuvre de la fermentation selon l'invention permet de maintenir la production de sophorolipides pendant au moins 672 heures de culture. La productivité volumique moyenne sur la durée de l'essai est de 1,38g.l⁻¹.h⁻¹ et le rendement massique moyen est de 0,43. Les performances du premier cycle sont plus faibles que la moyenne du fait que le temps de croissance de la souche y est plus long ; celles du cycle terminal sont en revanche plus élevées du fait que l'on récupère la totalité du moût contenu dans le fermenteur.

### EXEMPLE 2 (comparatif) :Culture discontinue alimentée ou "fed-batch" sans décantation du produit

On cultive, dans la même installation de fermentation, la souche selon le mode de culture du discontinu alimenté. L'alimentation en ester de colza est maintenue constante à 2,1 g par heure. On procède à des additions journalières de glucose de 100 g aux temps 24, 48, 72, 96, 120, 144 heures. Après 144 heures de culture, la vitesse d'assimilation du glucose se ralentit progressivement. On arrête la fermentation après 192 heures, lorsque la production de sophorolipides a cessé. Les performances de cet essai sont les suivantes (Tableau 2):

**Tableau 2 :**

| Performances de la fermentation discontinue alimentée (fed-batch) | | | | |
|---|---|---|---|---|
| Durée (h) | sophorolipides anhydres (g) | Acides gras dans sophorolipides (%) | Productivités (g.l⁻¹.h⁻¹) | Rendements |
| 192 | 630 | 0,5 | 1,64 | 0,52 |

Cet exemple montre que la culture de la souche selon le mode discontinu alimenté ou "fed-batch" ne permet pas de maintenir l'excrétion de sophorolipides au-delà d'une durée de culture sensiblement égale à 190 heures.

### EXEMPLE 3 (comparatif) : Culture continue en chémostat multi-étagé

On utilise une installation de cinq fermenteurs fonctionnant en série. Le premier a un volume utile de 0,44 litre tandis que les quatre autres ont un volume utile de deux litres. On alimente le premier en ester éthylique de colza à raison de 2,1 g par heure. On propage la souche dans le premier réacteur pendant 24 h (durée de la croissance) puis on lance la culture continue multi-étagée en alimentant le premier réacteur avec un flux de milieu frais (à 100 g.l⁻¹ de glucose) de 44 ml.h⁻¹. On récupère, à la température de 4°C pendant des périodes de 96 heures, le moût sortant du dernier fermenteur. Les performances de l'essai en chémostat multi-étagé sont indiquées dans le tableau 3.

**Tableau 3 :**

| Performances de la culture continue en chémostat multi-étagé | | | | | |
|---|---|---|---|---|---|
| Collecte | - Période | sophorolipides anhydres (g) | Acides gras dans sophorolipides (%) | Productivités (g.l⁻¹.h⁻¹) | Rendements |
| 1 | 0 à 96 h | 88 | 0,8 | 0,11 | 0,1 |
| 2 | 96 à 192 h | 31 | 3 | 0,04 | 0,5 |
| 3 | 192 à 288 h | 5 | 7,8 | 0,01 | 0,01 |
| 4 | 288 à 384 h | <5 | | <0,01 | <0,01 |
| 5 | 384 à 480 h | <5 | | <0,01 | <0,01 |
| 6 | 480 à 576 h | <5 | | <0,01 | <0,01 |
| 7 | 576 à 672 h | <5 | | <0,01 | <0,01 |

Cet exemple montre que lorsque l'on cultive la souche en continu selon le mode du chémostat, celle-ci se maintient dans le milieu de culture mais perd progressivement sa capacité d'excrétion de sophorolipdes.

### EXEMPLE 4: Culture cyclique avec alimentation discontinue et sans décantation du produit

On répète l'exemple 1 en remplaçant l'addition continue d'ester de colza par des additions ponctuelles d'ester en début de chaque cycle égales à 201,6 g. Les performances de l'essai sont rassemblées dans le tableau 4.

**Tableau 4 :**

| Performances de la fermentation cyclique avec alimentation discontinue d'ester et sans décantation du produit | | | | | |
|---|---|---|---|---|---|
| Cycles | Heures des prélèvements | sophorolipides anhydres (g) | Acides gras dans sophorolipides (%) | Productivités (g.l⁻¹.h⁻¹) | Rendements |
| 1 | 96 | 70 | 2,5 | 0,36 | 0,1 |
| 2 | 192 | 220 | 2,9 | 1,14 | 0,36 |
| 3 | 288 | 185 | 2 | 0,96 | 0,3 |
| 4 | 384 | 215 | 3,1 | 1,19 | 0,35 |
| 5 | 480 | 195 | 2,9 | 1,01 | 0,32 |
| 6 | 576 | 185 | 3,5 | 0,96 | 0,3 |
| 7 | 672 | 475 | 3 | 2,47 | 0,78 |
| Moyennes | | 220 | 2,8 | 1,15 | 0,35 |

Cet exemple montre que la culture cyclique avec alimentation discontinue de l'ester éthylique de colza produit plus de sophorolipides qu'en fed-batch mais moins de sophorolipides qu'en culture cyclique avec alimentation continue et sans décantation du produit. De plus, les sophorolipides récupérés renferment davantage d'impuretés que dans le cas de la culture cyclique avec alimentation continue d'ester de colza.

### EXEMPLE 5 : (selon l'invention) Culture cyclique avec alimentation continue et décantation des sophorolipides

On répète l'exemple 1 en modifiant chaque cycle de la façon suivante :

Avant de procéder à la récupération du moût de fermentation, on le laisser décanter pendant une à deux minutes. On récupère la totalité des sophorolipides décantés et une partie du moût de fermentation de façon à ne laisser dans le fermenteur qu'un litre de moût débarrassé de sophorolipides. Les résultats de l'essai sont rassemblés dans le tableau 5.

**Tableau 5 :**

| Performances de la fermentation cyclique avec alimentation continue d'ester et avec décantation du produit | | | | | |
|---|---|---|---|---|---|
| Cycles | Heures des prélèvements | sophorolipides anhydres (g) | Acides gras dans sophorolipides (%) | Productivités (g.l⁻¹.h⁻¹) | Rendements |
| 1 | 96 | 235 | <0,5 | 1,22 | 0,33 |
| 2 | 192 | 380 | <0,5 | 1,98 | 0,63 |
| 3 | 288 | 345 | <0,5 | 1,79 | 0,57 |
| 4 | 384 | 360 | <0,5 | 1,87 | 0,59 |
| 5 | 480 | 330 | <0,5 | 1,71 | 0,54 |
| 6 | 576 | 355 | <0,5 | 1,85 | 0,59 |
| 7 | 672 | 540 | <0,5 | 2,81 | 0,89 |
| Moyennes | | 363 | <0,5 | 1,89 | 0,59 |

Cet exemple montre que, conformément à l'invention, la mise en oeuvre d'une décantation des sophorolipides et leur récupération à la fin de chaque cycle permet d'obtenir un produit très peu contaminé par des acides gras. La productivité et le rendement de la fermentation conduite dans ces conditions sont plus élevés que dans le cas de la culture cyclique avec alimentation continue sans décantation du produit.

### EXEMPLE 6: Culture cyclique avec alimentation discontinue et décantation des sophorolipides

On répète l'exemple 4 en procédant à la fin de chaque cycle à la décantation et à la récupération des sophorolipides produits. Les résultats de l'essai sont indiqués dans le tableau 6.

**Tableau 6 :**

| Performances de la fermentation cyclique avec alimentation discontinue d'ester et avec décantation du produit | | | | | |
|---|---|---|---|---|---|
| Cycles | Heures des prélèvements | sophorolipides anhydres (g) | Acides gras dans sophorolipides (%) | Productivités (g.l⁻¹.h⁻¹) | Rendements |
| 1 | 96 | 225 | 1 | 1,17 | 0,32 |
| 2 | 192 | 360 | 1,3 | 1,87 | 0,59 |
| 3 | 288 | 350 | 1,7 | 1,82 | 0,58 |
| 4 | 384 | 345 | 0,9 | 1,79 | 0,57 |
| 5 | 480 | 330 | 1,1 | 1,71 | 0,54 |
| 6 | 576 | 345 | 1,3 | 1,79 | 0,57 |
| 7 | 672 | 515 | 2 | 2,68 | 0,85 |
| Moyennes | | 352 | | 1,83 | 0,57 |

Cet exemple montre que la productivité et le rendement de la fermentation cyclique avec alimentation discontinue et décantation des sophorolipides sont élevés. Cependant, la pureté des sophorolipides est moins bonne, ce qui nécessitera des extractions successives par solvant, à chaud de préférence, pour atteindre un niveau de pureté comparable à celui du produit de l'exemple 5.

## Revendications

1. Procédé de production d'une composition de sophorolipides comprenant la mise en culture dans une zone de réaction dans des conditions de culture aérée d'une souche *Candida bombicola* ou *Candida apicola* dans un milieu contenant une source d'azote, et un substrat, le procédé étant **caractérisé en ce qu'**il comprend un premier cycle de fermentation comportant une étape de croissance de la souche et une étape de production d'un moût de fermentation contenant des sophorolipides au moyen d'une alimentation de la souche en substrat, dans la zone de réaction, le procédé étant en outre **caractérisé en ce qu'**on effectue au moins une fois les étapes suivantes :
a) on soutire une partie du moût de fermentation;
b) on ajoute à la partie restante du moût un milieu minéral contenant de l'azote de façon à constituer un second milieu;
c) on effectue un nouveau cycle de fermentation à partir de ce second milieu et on produit un autre moût de fermentation ; la durée des dits premier et nouveau cycles de fermentation étant d'au moins 30 heures et de préférence d'au moins 70 heures, le procédé étant de plus **caractérisé en ce que** l'on récupère les sophorolipides du moût des étapes (a) et (c).

2. Procédé selon la revendication 1 dans lequel on sépare le moût de fermentation produit et on récupère un moût de fermentation débarrassé au moins en partie des sophorolipides sur lequel on effectue les étapes (a) à (c).

3. Procédé selon l'une des revendications 1 ou 2, dans lequel on ajoute, à la partie restante du moût, du milieu minéral en quantité sensiblement égale à la quantité prélevée.

4. Procédé selon l'une des revendications 1 à 3, dans lequel on soutire une quantité de moût telle qu'il reste au moins 5 % en poids et de préférence 20 à 40 % du moût dans la zone de réaction.

5. Procédé selon l'une des revendications 1 à 4, dans lequel on interrompt chaque cycle de fermentation lorsque la productivité en sophorolipides durant le dit cycle diminue et de préférence on l'interrompt avant 140 heures de fermentation et plus particulièrement après une période de temps comprise entre 70 et 120 heures.

6. Procédé selon l'une des revendications 1 à 5, dans lequel on stoppe le dernier cycle de fermentation, lorsque la productivité en sophorolipides calculée sur les 24 premières heures de production de ce dernier cycle atteint 70 % de la productivité du premier cycle calculé sur la même période de production.

7. Procédé selon l'une des revendications 1 à 6, dans lequel on maintient la concentration résiduelle en substrat dans la zone de réaction durant chaque cycle de fermentation à une valeur qui n'excède pas 18 grammes par litre de volume réactionnel initial pendant qu'on alimente la zone de réaction en substrat.

8. Procédé selon l'une des revendication 1 à 7, dans lequel le substrat généralement utilisé est au moins une huile animale, au moins une huile végétale et/ou au moins un ester de ladite huile, ladite huile ou ledit ester incorporant une chaîne linéaire aliphatique de 10 à 24 atomes de carbone.

9. Procédé selon l'une des revendications 1 à 8, dans lequel le milieu minéral de l'étape (b) et le milieu de culture contiennent un sucre.

10. Procédé selon l'une des revendications 1 à 9, dans lequel la souche est *Candida bombicola* CBS 6009.

11. Procédé selon l'une des revendications 1 à 10, dans lequel on alimente la souche en substrat de manière sensiblement continue, à un débit d'alimentation compris entre 0,01 et 4 g par heure et par litre de volume réactionnel initial.

## Claims

1. Process for the production of a composition of sophorolipids that includes the cultivation in a reaction zone under aerated cultivation conditions of a stock of **Candida bombicola** or **Candida apicola** in a medium that contains a source of nitrogen and a substrate, with the process being **characterized in that** it includes a first fermentation cycle that includes a stage of growth of the stock and a stage of production of a fermentation wort that contains sophorolipids with a feed of the stock by substrate, in the reaction zone, with the process also being **characterized in that** the following stages are carried out at least once:
a) a portion of the fermentation wort is drawn off;
b) a mineral medium that contains nitrogen is added to the remaining portion of the wort to constitute a second medium;
c) a new fermentation cycle is carried out from this second medium, and another wort is produced; with the duration of the first and new fermentation cycles being at least 30 hours and preferably at least 70 hours, and with the process also being **characterized in that** the sophorolipids are recovered from the wort of stages (a) and (c).

2. Process according to claim 1, wherein the fermentation wort that is produced is separated, and a fermentation wort that is freed of at least a portion of sophorolipids, on which stages (a) to (c) are carried out, is recovered.

3. Process according to one of claims 1 or 2, wherein to the remaining portion of the wort is added mineral medium in an amount that is approximately equal to the quantity removed.

4. Process according to one of claims 1 to 3, wherein a quantity of wort is drawn off such that there remains at least 5% by weight and preferably 20 to 40% by weight of wort in the reaction zone.

5. Process according to one of claims 1 to 4, wherein each fermentation cycle is interrupted when the sophorolipid productivity during the so-called cycle decreases, and preferably it is interrupted before 140 hours of fermentation and more particularly after a period of between 70 and 120 hours.

6. Process according to one of claims 1 to 5, wherein the last fermentation cycle is halted when the productivity of sophorolipids, calculated over the first 24 hours of production of this last cycle, reaches 70% of the productivity of the first cycle, calculated over the same production period.

7. Process according to one of claims 1 to 6, wherein during each fermentation cycle the residual concentration of substrate in the reaction zone is kept at a value that does not exceed 18 grams per liter of initial reaction volume while the reaction zone is fed with substrate.

8. Process according to one of claims 1 to 7, wherein the substrate that is generally used is at least one animal oil, at least one vegetable oil, and/or at least one ester of said oil, with said oil or said ester incorporating an aliphatic linear chain of 10 to 24 carbon atoms.

9. Process according to one of claims 1 to 8, wherein the mineral medium of stage (b) and the culture medium contain sugar.

10. Process according to one of claims 1 to 9, wherein the stock is **Candida bombicola CBS 6009.**

11. Process according to one of claims 1 to 10, wherein the stock is fed by substrate in an approximately continuous manner at a feed rate of between 0.01 and 4 g per hour and per liter of initial reaction volume.

## Patentansprüche

1. Verfahren zur Herstellung einer Zusammensetzung von Sophorolipiden, das die Kultivierung eines *Candida-bombicola-* oder *Candida-apicola*-Stammes in einer Reaktionszone unter aeroben Kulturbedingungen in einem Medium umfasst, das eine Stickstoffquelle und ein Substrat enthält, wobei das Verfahren **dadurch gekennzeichnet ist, dass** es einen ersten Fermentationszyklus umfasst, der eine Wachstumsstufe des Stammes und eine Stufe der Herstellung einer Sophorolipide enthaltenden Fermentationsmischung mittels einer Substratversorgung des Stammes in einer Reaktionszone umfasst, wobei das Verfahren im übrigen **dadurch gekennzeichnet ist, dass** wenigstens einmal die folgenden Stufen durchgeführt werden:
a. ein Teil der Fermentationsmischung wird abgezogen;
b. zu dem verbleibenden Teil der Mischung wird ein mineralisches stickstoffhaltiges Medium gegeben, um ein zweites Medium zu bilden;
c. ein neuer Fermentationszyklus wird ausgehend von dem zweiten Medium durchgeführt und eine andere Fermentationsmischung wird erzeugt; wobei die Dauer der ersten und neuen Fermentationszyklen wenigstens 30 Stunden, vorzugsweise wenigstens 70 Stunden beträgt, wobei das Verfahren zusätzlich **dadurch gekennzeichnet ist, dass** die Sophorolipide der Mischung der Stufen (a) und (c) gewonnen werden.

2. Verfahren nach Anspruch 1, bei dem die erzeugte Fermentationsmischung getrennt wird und eine wenigstens teilweise von den Sophorolipiden befreite Fermentationsmischung gewonnen wird, auf der die Stufen (a) bis (c) durchgeführt werden.

3. Verfahren nach einem der Ansprüche 1 oder 2, bei dem zu dem verbleibenden Teil der Mischung mineralisches Medium in einer Menge zugegeben wird, die im wesentlichen gleich der entnommenen Menge ist.

4. Verfahren nach einem der Ansprüche 1 bis 3, bei dem eine Menge an Mischung derart abgezogen wird, dass wenigstens 5 Gew.-% und vorzugsweise 20 bis 40 % der Mischung in der Reaktionszone verbleiben.

5. Verfahren nach einem der Ansprüche 1 bis 4, bei dem jeder Fermentationszyklus unterbrochen wird, wenn die Produktivität an Sophorolipiden während des Zyklus abnimmt, und vorzugsweise vor 140 Stunden Fermentation und insbesondere nach einem Zeitraum zwischen 70 und 120 Stunden unterbrochen wird.

6. Verfahren nach einem der Ansprüche 1 bis 5, bei dem der letzte Fermentationszyklus gestoppt wird, wenn die über die ersten 24 Produktionsstunden errechnete Produktivität an Sophorolipiden dieses letzten Zyklus 70% der Produktivität des ersten im gleichen Produktionszeitraum berechneten Zyklus erreicht.

7. Verfahren nach einem der Ansprüche 1 bis 6, bei dem die Restkonzentration an Substrat in der Reaktionszone während jedes Fermentationszyklus bei einem Wert, der 18 Gramm pro Liter anfänglichen Reaktionsvolumens nicht überschreitet, beibehalten wird während die Reaktionszone mit Substrat versorgt wird.

8. Verfahren nach einem der Ansprüche 1 bis 7, bei dem das im allgemeinen verwendete Substrat wenigstens ein tierisches Öl, wenigstens ein pflanzliches Öl und/oder wenigstens ein Ester dieses Öles ist, wobei das Öl oder der Ester eine lineare aliphatische Kette von 10 bis 24 Kohlenstorfatomen enthält.

9. Verfahren nach einem der Ansprüche 1 bis 8, bei dem das mineralische Medium der Stufe (b) und das Kulturmedium Zucker enthalten.

10. Verfahren nach einem der Ansprüche 1 bis 9, bei dem der *Candidabombicola*-Stamm CBS 6009 ist.

11. Verfahren nach einem der Ansprüche 1 bis 10, bei dem man den Stamm in im wesentlichen kontinuierlicher Weise bei einem Versorgungsdurchsatz zwischen 0,01 und 4 g pro Stunde und pro Liter anfänglichen Reaktionsvolumens mit Substrat versorgt.
